# EUROPEAN PATENT SPECIFICATION

(11) **EP 4 574 120 B1**
(45) Date of publication and mention of the grant of the patent: **18.11.2026**
(21) Application number: 23218461.4
(22) Date of filing: 20.12.2023
(51) Int. Cl.: A61F 13/537

(54) **WASHABLE ABSORBENT ASSEMBLY**
WASCHBARE SAUGFÄHIGE ANORDNUNG
ENSEMBLE ABSORBANT LAVABLE

(43) Date of publication of application: 25.06.2025
(73) Proprietor: Essity Hygiene and Health Aktiebolag, 405 03 Göteborg (SE)
(72) Inventor: VASELL, Anna, 405 03 GÖTEBORG (SE); KALENTUN, Pia, 405 03 GÖTEBORG (SE); HAGSON, Sandra, 405 03 GÖTEBORG (SE); LEANDER, Maja, 405 03 GÖTEBORG (SE)
(74) Representative: Nederlandsch Octrooibureau

(56) References cited:
- EP-B1- 2 938 309
- US-A1- 2023 201 048
- US-A1- 2023 248 585

## Description

### TECHNICAL FIELD

The disclosure pertains to a washable absorbent assembly adapted to be permanently or detachably secured in an outer textile pant. Furthermore, the disclosure pertains to a washable absorbent hygienic underwear comprising an outer textile pant wherein the absorbent assembly being permanently secured in the outer textile pant, and to a washable absorbent assembly being a washable pad adapted to be detachably secured in an outer textile pant. The absorbent assembly comprising a liquid permeable layer, a liquid impermeable layer and an absorbent member located between the liquid permeable layer and the liquid impermeable layer.

### BACKGROUND

Washable and reusable absorbent hygienic underwear and pads as disclosed herein are intended for use by persons leading a normal socially and physically active life and suffering from light to moderate urine incontinence, so from 1 ml to about 100 ml body liquid, such as for example urine.

It is a concern to provide such washable absorbent underwear and pad which as much as possible resemble ordinary underwear and which are comfortable, well-fitting and can be inconspicuously worn under normal clothing and yet provide adequate urine absorbency and leakage security. By necessity, washable absorbent underwear includes layers and materials which are not present in conventional underwear, such as a liquid permeable top layer which lets liquid pass through into an underlying liquid absorbing member. It is also generally preferred that a liquid barrier layer is arranged on a garment-facing side of the layer or layers of the absorbent member to prevent liquid from escaping through the incontinence protection underwear and to wet the user's outer garment.

EP2938309B1 discloses a disposable absorbent article. Unlike disposable incontinence articles, washable incontinence garments do generally not contain superabsorbent material, which renders the liquid retention capacity of such articles less than the liquid retention capacity of a comparable superabsorbent containing disposable article. To merely increase the amount of absorbent material in a washable absorbent underwear would be directly contra-productive to the underwear-likeness of the garment as it would make the garment bulky and less conformable and comfortable than desired. Washable sanitary articles for absorbing menstrual discharge are known from US 2023/0201048. A reusable outer cover for an absorbent article is known from US 2023248585.

An object of the present disclosure is to offer an improved washable absorbent hygienic underwear or pad, having adequate absorbency and leak protection properties for light to moderate urine incontinence as well as an improved likeness to ordinary underwear.

### SUMMARY

The above and further objects may be achieved with a washable absorbent assembly adapted to being permanently or detachably secured in an outer textile pant in accordance with claim 1. Variations of the disclosure are set out in the dependent claims and in the following description.

The disclosure concerns a washable absorbent assembly adapted to be permanently or detachably secured in an outer textile pant (10) and having an extension in a longitudinal direction (y), in a transverse direction (x) and in a thickness direction (z) perpendicular to the longitudinal direction (y), and the transverse direction (x), and comprising:
- a liquid permeable layer (120),
- a liquid impermeable layer (150); and
- an absorbent member (140) located between the liquid permeable layer (120) and the liquid impermeable layer (150), the absorbent member (140) comprising a liquid acquisition structure (141) and a liquid retaining structure (142), the liquid acquisition structure (141) being arranged between the liquid permeable layer (120) and the liquid retaining structure (142), wherein

the liquid permeable layer (120) has a basis weight of less than 300 g/m²;
the liquid acquisition structure (141) comprises or consists of a spacer fabric (138) comprising a top web (138a), a bottom web (138b) and an intermediate section (138c) comprising pile filaments extending in the thickness direction (z) and connecting the top web (138a) with the bottom web (138b), the spacer fabric (138) having a density of 0.1 g/cm³ or less at an applied pressure of 0.5 k Pa, the liquid retaining structure (142) has a higher density than the liquid acquisition structure (141) and the washable absorbent assembly is devoid of superabsorbent particles.

The difference in density between the liquid acquisition structure and the liquid retaining structure in the absorbent assembly ascertains that the preferential liquid transfer direction between the structures is from the liquid acquisition structure to the liquid retaining structure.

Furthermore, the disclosure concerns a washable absorbent underwear, wherein the absorbent assembly is being permanently secured in a pant (10). The pant (10) is preferably a textile pant (10).

Furthermore, the disclosure concerns a washable absorbent pad, wherein the washable absorbent pad is the absorbent assembly being adapted to be detachably secured in a user's underwear.

In the washable absorbent assembly as disclosed herein, the density of the liquid retaining structure may be higher than the density of the spacer fabric in the liquid acquisition structure. The density of the liquid retaining structure may be 20% higher than the density of the spacer fabric or more than 50%, or more than 80% higher than the density of the spacer fabric.

The liquid acquisition structure drains liquid from the liquid permeable layer and acts as a temporary reservoir for the liquid until it has been absorbed by the underlying liquid retaining structure. The liquid may be distributed in the x and y directions by flowing in the internal space of the spacer fabric. In the higher density liquid retaining structure, liquid distribution is to a higher degree dependent on wicking in the capillaries formed between fibers in the structure. Due to the density difference, transportation of liquid from the high-bulk/low density liquid acquisition structure takes place quickly after a liquid insult, which means that the liquid acquisition structure is restored to an air-filled lofty state within a few seconds, after wetting and is made ready for a new liquid insult. In addition, the liquid acquisition structure can act as an air-filled, pressure-resistant distance element between the liquid which is absorbed in the liquid retaining structure and a wearer's body. This is beneficial both as it provides the absorbent assembly in the underwear with a less wet and clammy feeling and as the air-filled space in the spacer fabric counteracts liquid return to the liquid permeable layer. In the washable absorbent assembly as disclosed herein, the liquid acquisition structure may consist of a single layer of spacer fabric or of a layer of spacer fabric and one or more additional material layers. The liquid acquisition structure may contain further layers in addition to the spacer fabric layer, such as a second spacer fabric layer and/or an open-mesh material arranged between top layer and the spacer fabric.

The spacer fabric in the liquid acquisition structure is a relatively stiff and non-compressible material. A high resistance to compression in the spacer fabric is beneficial as it allows the liquid acquisition structure to retain a large liquid-receiving and liquid containing space within the internal network formed by the pile filaments connecting the top and bottom layers of the spacer fabric, even when exposed to the compressive forces which normally arise during use of the underwear.

The spacer fabric in the liquid acquisition structure of the washable absorbent underwear may have a bulk of 10 cm³/g or more at an applied pressure of 5 kPa. The bulk value is the inversion of the density value, so a spacer fabric with a bulk of 10 cm³/g at an applied pressure of 5 kPa, has a density of 0.10 g/cm³ at an applied pressure of 5 kPa.

Also, the spacer fabric in the liquid acquisition structure may have a bulk of 8 cm³/g or more at an applied pressure of 10 kPa. Furthermore, at an applied pressure of 20 kPa, the bulk may be 5 cm³/g or more.

The spacer fabric in the liquid acquisition structure is a relatively stiff and non-extensible material. As disclosed herein, a high resistance to compression of the spacer fabric is beneficial as it allows the liquid acquisition structure to retain a liquid-receiving and liquid containing space within the internal network formed by the pile filaments in the intermediate section of the spacer fabric between the outer layers, even when exposed to the compressive forces which normally arise during use of the underwear.

In the washable absorbent assembly as disclosed herein, the liquid retaining structure may consist of a single material layer or of 2 or more individual material layers. If the liquid retaining structure contains more than one layer of textile absorbent material, it may be beneficial that the layers provide a density gradient in the z-direction, with increasing density in the layers of the liquid retaining structure from a top layer facing the liquid permeable layer to a bottom layer facing the liquid impermeable layer.

The spacer fabric may have a thickness of 2 mm or more. The spacer fabric may have a relatively low basis weight, such as 150 g/m² to 300 g/m² or 170 g/m² to 270 g/m². The basis weight of the liquid retaining structure may preferably be higher than the basis weight of the liquid acquisition structure.

In the liquid acquisition structure, the layer of spacer fabric may be in direct contact with the liquid permeable layer or may be in indirect contact with the liquid permeable layer by means of an intervening layer such as a reticulate textile layer or similar.

In the washable absorbent assembly as disclosed herein, the layer of spacer fabric may be in direct contact with the liquid retaining structure. Such arrangement may be beneficial as it promotes liquid transfer from the spacer fabric to the liquid retaining structure and facilitates rapid draining of the spacer fabric after a liquid insult.

In the washable absorbent assembly as disclosed herein, a density of the bottom web of the spacer fabric may be higher than a density of the top web of the spacer fabric. A higher density in the bottom web of the spacer fabric may promote liquid draining, away from the top web and further into the liquid retaining structure.

The spacer fabric may have a bulk of 10 g/cm³ or more at an applied pressure of 5 kPa.

The layer of spacer fabric may be in direct contact with the liquid permeable layer or may be in indirect contact with the liquid permeable layer by means of an intervening layer such as a reticulate textile layer or similar.

The "density of the pile filament" is defined as the number of pile filament connections per cm². The density of pile filament connections may be 50-150/cm², preferably 70-100/cm². The pile filaments may have a fineness of 80-130 dtex, preferably 90-120 dtex. The pile filaments may be of a monofilament yarn leading to a high compressive strength while keeping a low grammage.

The spacer fabric may have 5-15, preferably 8-12 courses/cm in the production direction of the fabric and 6-12, preferably 7-10 wales/cm in the cross direction of the fabric.

The bottom layer of the spacer fabric may have a denser structure with smaller openings than the top layer.

In the washable absorbent assembly as disclosed herein, the pile filaments extending in the thickness direction z between the top web and the bottom web form the intermediate section of the spacer fabric, the density (g/cm³) of the top web being higher than a density of the intermediate section. A relatively higher density in the top web of the spacer fabric may promote draining of liquid from the liquid permeable layer. Similarly, it may be beneficial that the density of the bottom web of the spacer fabric is higher than the density of the intermediate section of the spacer fabric, promoting draining of the intermediate section of the spacer fabric into the bottom web of the spacer fabric. Further, the density of the bottom web of the spacer fabric is preferably lower than the density of an adjoining layer of the liquid retaining structure to promote liquid draining from the bottom web of the spacer fabric into the liquid retaining structure.

The layers of the absorbent assembly may be joined by means of seaming or welding. Joining seams are preferably flat seams, creating a smooth transition between material layers. The use of flat seams contributes to making the washable absorbent hygienic underwear as comfortable as possible.

In the washable absorbent assembly as disclosed herein, the liquid retaining structure may be attached to the liquid impermeable layer, e.g., by lamination. The combined components forms an absorbent liquid impermeable composite which may be handled as a single component when manufacturing the washable absorbent assembly.

A washable absorbent underwear as disclosed herein may be a washable absorbent hygienic underwear for male users, such as boxer shorts or briefs. In washable absorbent male underwear, the absorbent structure is typically placed with all or at least a major part of the absorbent material in the front portion of the underwear. The liquid acquisition structure and optionally also the liquid retaining structure may have a generally triangular shape in the x-y plane with the base of the triangle aligned with the waistline of the underwear and the tip of the triangle directed downward in the y-direction towards the crotch portion of the washable absorbent underwear. The liquid retaining structure may be arranged to provide supplementary absorbent material in areas of the washable absorbent underwear which may be subject to wetting but which are not part of a primary wetting and absorption zone of the washable absorbent underwear which is covered by the liquid acquisition structure. Such areas may include portions of the washable absorbent underwear located in the x-direction outside the primary wetting zone in the waist area of the washable absorbent underwear and/or a portion of the washable absorbent underwear located in the crotch portion of the washable absorbent underwear.

Alternatively, the washable absorbent underwear as disclosed herein may be washable absorbent underwear for female users, such as female underpants. In washable absorbent female underwear, the absorbent structure is typically placed with a major part of the absorbent material in the crotch portion and the lower front portion of the underwear where initial wetting of the washable absorbent underwear is most likely to take place.

Furthermore, the washable absorbent underwear as disclosed herein may be a unisex washable absorbent underwear.

The liquid retaining structure in the absorbent assembly may have a larger area than the liquid acquisition structure in the absorbent assembly and may be used to provide additional absorbency in areas of the washable absorbent underwear which are outside a primary wetting and absorption zone of the washable absorbent underwear, but which may still be subject to wetting. The liquid acquisition structure in the absorbent assembly may completely overlap with the liquid retaining structure and have an area which is 65% or less of the area of the liquid retaining structure.

Alternatively, the liquid acquisition structure in the absorbent assembly may have a larger area than the liquid retaining structure in the absorbent assembly. The liquid retaining structure in the absorbent assembly may completely overlap with the liquid acquisition structure and have an area which is 65% or less of the area of the liquid acquisition structure.

*By washable* as defined herein is implied an ability of the absorbent hygienic underwear to withstand laundering at 40 °C at least 100 times without any appreciable loss in appearance, integrity and/or functionality. When used herein laundering means that the absorbent underwear may be subjected to an aqueous solution containing detergent.

As used herein, the term *washable absorbent underwear* refers to garments which are worn as a pair of underpants and which are intended to be placed against the skin of the wearer to absorb and contain body fluids such as e g urine and /or menses. The underwear as disclosed herein is an undergarment, or underpant intended for use by persons suffering from light to moderate incontinence. The absorbent underwear is a fabric underwear, also referred to as a textile underwear, and is not a diaper. The underwear according to the present disclosure is intended to be worn in the same manner as conventional underwear and to be laundered after use for reuse as an absorbent incontinent protection underwear.

The washable absorbent hygienic underwear may be a washable absorbent incontinence underwear.

The term *washable absorbent pad* refers to a washable absorbent assembly adapted to be detachably secured in a textile pant. The washable absorbent pad may be a washable absorbent pad for female users or male users.

The term *fabric* as used in the present disclosure refer to webs of single or multiple layers of textile materials. The fabric may be knitted or woven wherein knitting is a method of constructing a fabric by interlocking a series of loops of one or more yarns. There are two major classes of knitting namely warp and weft knitting. Weaving is a method or process of interlacing yarns so that they cross each other at right angles to produce woven fabric. The warp yarns run lengthwise in the fabric and the filling threads (weft) or picks, run from side to side.

The term *fiber* is used herein in a broad sense and includes fibers which have a measurable length, e.g., staple fibers, as well as filaments which do not have a measurable length and which are often referred to as being *"endless".* The term *fiber* is also intended to cover microfibers and fibrils. The fibers in the textile materials which are used in the washable absorbent underwear as disclosed herein may be spun into yarns or threads each yarn or thread consisting of multiple fibers.

When used herein a *permanent attachment,* refers to an attachment which is intended to remain intact and not be broken before, during or after use of the washable absorbent hygienic underwear as disclosed herein. A permanently attached absorbent assembly is an absorbent assembly which cannot be separated from the textile pant of the washable absorbent hygienic underwear without breaking or otherwise destroying the washable absorbent underwear. The absorbent assembly is not necessarily directly bonded to the textile pant but may instead be attached via leg opening seams or bonded by an intermediate means such as a sealing tape.

The washable absorbent assembly contains no superabsorbent particles. However, the washable absorbent assembly may contain superabsorbent fibers in the liquid retention structure.

According to an alternative embodiment, the washable absorbent assembly comprises neither superabsorbent particles nor superabsorbent fibres. According to an embodiment, the washable absorbent assembly does not contain superabsorbent material of any kind.

The spacer fabric may contain a proportion of absorbent cellulosic fibers in the top and bottom layers or may be free or substantially free from absorbing fibres. The spacer may not contain superabsorbent material of any kind. It has further been found that in washable textile absorbent hygienic underwear which does not include superabsorbent material, the liquid has a higher tendency to transfer downwards under the influence of gravity. This means that the risk for leakage below a superabsorbent free absorbent core placed in a front portion of the incontinence product is higher than in a superabsorbent containing product.

The liquid permeable layer in the washable absorbent underwear as disclosed herein may have a basis weight of 300 g/m² or less, such as a basis weight of less than 200 g/m² or a basis weight or 150 g/m² or less. The liquid permeable layer may have a basis weight between 50 g/m² and 300 g/m².

In the washable absorbent underwear as disclosed herein, the liquid retaining capacity of the liquid spacer fabric may be lower than the liquid retaining capacity in the liquid retaining structure. The liquid retaining capacity of the liquid spacer fabric may be 0.2 g/cm², or less and the liquid retaining capacity in the liquid retaining structure may be 0.3 g/cm² or more.

A particularly efficient combination of comfort properties and absorption and leakage protection properties has been found to be achieved with an absorbent assembly having the following features in combination: a liquid permeable layer having a basis weight of 300 g/m² or less, a liquid acquisition structure comprising a spacer fabric having a density of 0.1 g/cm³ or less, a liquid retaining capacity in the spacer fabric of 0.2 g/cm² or less and a liquid retaining capacity in the absorbent retaining structure of 0.3 g/cm² or more. The density of the liquid retaining structure may be 20% higher than the density of the liquid acquisition structure. The density of the liquid retaining structure may be 50% higher than the density of the spacer fabric in the liquid acquisition structure.

The absorbent assembly comprises the following components, as seen in a direction from an inner wearer-facing surface of the washable absorbent hygienic underwear towards an outer garment facing surface of the washable absorbent hygienic underwear:

### A liquid permeable layer

The liquid permeable layer is a liquid wicking and transfer layer. The liquid permeable layer is preferably a hydrophilic layer which can attract aqueous liquid and let the liquid pass through the layer to underlying layers of the absorbent assembly, such as an underlying liquid acquisition layer which can drain liquid from the liquid permeable layer.

The liquid permeable layer may be made from any suitable material which can withstand repeated washing. Suitable textile materials include fibrous knitted or woven materials, such as tricot materials, as well as mesh materials, such as polymer netting, perforated plastic materials, and the like.

The layer may comprise or consist of natural fibers such as cotton, regenerated cellulose fibers such as viscose fibers or synthetic fibers such as polyester fibers, polyolefin fibers, etc. The synthetic fibers may be mono-, bi-, or multi-component fibers. Different types of fibers may be combined in the layer, e.g., to promote liquid wicking through the layer while at the same time retaining a dry surface feeling. It may be preferred that the liquid permeable layer comprises at least a portion of inherently wettable (hydrophilic) fibers which may promote liquid uptake and wicking in the layer. The liquid permeable layer may comprise at least a portion of synthetic fibers which do not absorb or retain liquid. The layer may, for example, comprise or consist of a combination of viscose or other regenerated fibers and polyolefin fibers, such as polypropylene fibers. However, liquid permeable layers made exclusively of natural or regenerated cellulosic fibers as well as liquid permeable layers made exclusively of synthetic fibers are also contemplated for the washable absorbent hygienic underwear as disclosed herein. Unless having a great open area, a liquid permeable layer made exclusively from synthetic, inherently non-wettable fibers will generally be treated, such as by a surfactant, to render the outer surface of the fibers hydrophilic and wettable.

### A liquid acquisition structure

The liquid acquisition structure drains liquid from the liquid permeable layer, distributes liquid in the liquid acquisition structure and transfers liquid to an underlying liquid absorbing layer, such as a liquid retaining structure. The liquid acquisition structure preferably has a high bulk and an internal volume which allows liquid to be received and temporarily held in the layer. The liquid acquisition structure may further serve as a spacer element, isolating liquid from a wearer's skin and preventing absorbed liquid from returning to the liquid permeable layer also when the washable absorbent hygienic underwear is exposed to pressure during use.

The liquid acquisition structure may be composed of one or more layers of woven, knitted, or nonwoven textile material, such as a three-dimensional spacer material, a terry cloth material or washable nonwoven material.

As disclosed herein, the liquid acquisition structure used in the washable absorbent hygienic underwear may preferably comprise or consist of a spacer fabric. The spacer fabric comprises two outer layers, such as two tricot layers which are connected to each other by spacer elements extending generally perpendicular between the layers. The spacer elements are formed from pile filaments which are relatively bending resistant and resilient as compared to the fibers in the outer layers.

The spacer fabric may be produced by knitting, as an integral structure. The outer layers may comprise wettable, absorbent fibers, such as natural or regenerated cellulosic fibers and the spacer elements may be made from synthetic material which will retain its resiliency in a wet state.

When the washable absorbent hygienic underwear or pad is being used, body fluids such as urine and/or menses which passes through the liquid permeable layer and into the liquid acquisition structure may be absorbed by the outer layers of the spacer fabric. The non-absorbent pile filaments create a liquid receiving and containing space between the outer layers of the spacer fabric. The liquid receiving and containing space is also referred to herein as an intermediate section of the spacer fabric. The liquid containing space may serve as a reservoir for liquid which may be subsequently absorbed by the lower of the two outer layers and of further absorbent material arranged below the liquid acquisition structure. Liquid may run relatively freely within the space of the channel network formed by the pile filaments between the outer layers of the spacer material, allowing the liquid to spread out over a greater area of the absorbent structure and increasing the area of the absorbent structure over which liquid transfer to a lower layer or layers may take place.

### A liquid retaining structure

The liquid retaining structure may be composed of one or more layers of woven, knitted, or nonwoven textile material arranged to receive liquid from the liquid acquisition structure.

The liquid retaining structure may be made from a three-dimensional spacer material, a terry cloth material or washable nonwoven material. The liquid retaining structure preferably has a higher ability of absorbing and retaining liquid in the structure than the liquid acquisition structure and is able to drain liquid from the liquid acquisition structure.

The liquid retaining structure may comprise synthetic fibers, natural fibers or man-made cellulosic fibers as set out above for the liquid acquisition structure. The liquid retaining structure may contain super absorbent fibers. The liquid retaining structure may comprise any blend or combination of suitable fibers.

### A liquid impermeable layer

The liquid impermeable layer serves to keep liquid in the absorbent member of the absorbent assembly and to prevent liquid from leaking out through the textile pant or being absorbed by the textile pant material.

The liquid impermeable layer is typically a polymer film layer or membrane. The polymer film is preferably breathable, letting air and water vapor pass through the film but forming a liquid barrier. A breathable polyurethane film may be used for the liquid impermeable layer. The liquid impermeable layer may be directly attached to an absorbent layer, such as an absorbent layer in the liquid retaining structure. The attachment may be e.g., by a seam or by lamination. Lamination of the liquid impermeable layer to the liquid retaining structure may be made by means of a construction adhesive or by extruding the liquid permeable layer onto the absorbent layer of the liquid retaining structure. The construction adhesive may be e.g., a polyurethane based adhesive.

The washable absorbent assembly may be a washable absorbent pad adapted to be detachably secured to a textile pant.

The present disclosure also concerns a washable absorbent hygienic underwear comprising an absorbent assembly according to any of claims 1 to 19, and a textile pant, wherein the absorbent assembly is permanently secured in the textile pant. The absorbent hygienic underwear having an extension in a longitudinal direction (y), in a transverse direction (x) and in a thickness direction (z) perpendicular to the longitudinal direction (y), and the transverse direction (x), and the textile pant comprising a front portion, a rear portion and a crotch portion. The washable absorbent hygienic underwear may be a washable absorbent incontinence underwear.

The material in the textile pant is preferably a tricot material such as a cotton tricot material comprising elastic material such as elastane. The washable absorbent hygienic underwear is preferably provided with a separately applied elastic waistband. The elastic waistband may comprise the same tricot material as the outer pant material, or any other suitable textile material. The elastic waistband preferably comprises waist elastic elements, such as elastic threads, or one or more elastic bands.

In a washable absorbent underwear, the liquid permeable layer, the absorbent layer or layers and the liquid impermeable layer of the absorbent assembly may be attached to the textile pant by means of seaming. A liquid impermeable tape may be applied around the peripheral edges of the absorbent assembly.

### BRIEF DESCRIPTION OF THE DRAWINGS

The washable absorbent assembly will be further explained hereinafter by means of nonlimiting examples and with reference to the appended drawings wherein:
- Fig. 1: shows a perspective frontal view of absorbent underwear for female users;
- Fig. 2: shows the absorbent underwear of Fig. 1, from an inner, wearer-facing side, and in a flat-out state with open side seams;
- Fig. 3: shows a cross section taken along the line III-III in Fig. 2; and
- Fig. 4: shows a perspective frontal view of absorbent underwear for male users; and
- Fig. 5: shows a perspective view of an exemplary spacer fabric.

### DETAILED DESCRIPTION

It is to be understood that the drawings are schematic and that individual components or features, such as layers of material are not necessarily drawn to scale.

The absorbent hygienic underwear shown in the figures is provided as an example only and should not be considered limiting to the invention as disclosed herein. Although the washable absorbent assembly shown in the figures is permanently secured to a washable absorbent underwear, it is to be understood that the washable absorbent assembly may be a washable absorbent pad being detachably secured to a textile pant.

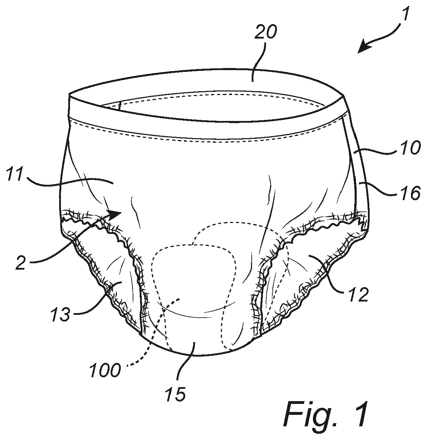

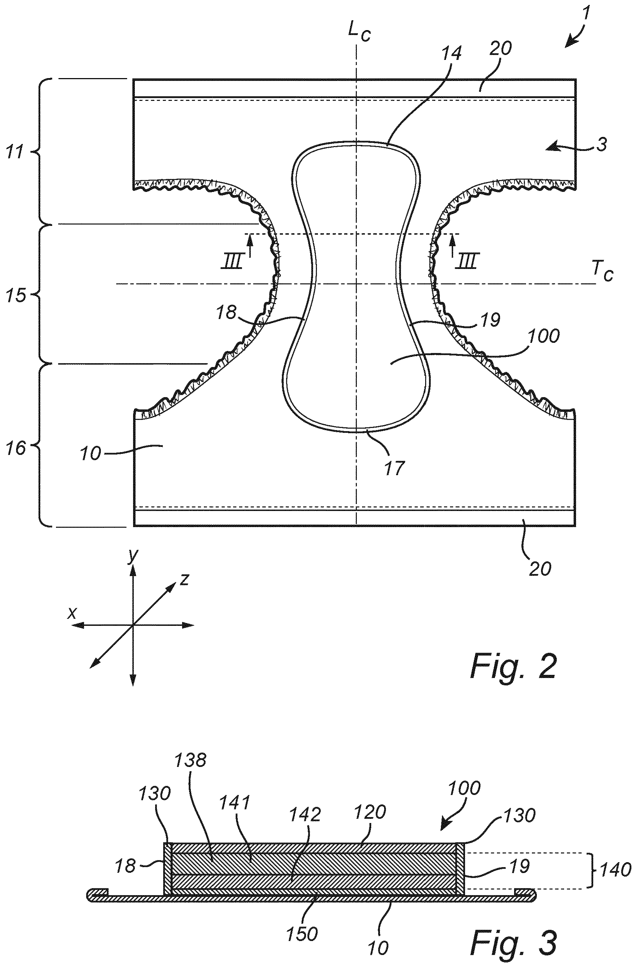

The washable absorbent hygienic underwear disclosed herein should not be construed as being limited to the aspects set forth herein but can be varied within the scope of the appended claims. Although the washable absorbent hygienic underwear shown in Figs. 1 and 2 takes the form of incontinence panties for female users, it is to be understood that the washable absorbent underwear may be incontinence underwear for male users or may be unisex incontinence underwear. The washable absorbent underwear may be of any useful design, such as panties, briefs, boxer-shorts, etc., as known in the art.

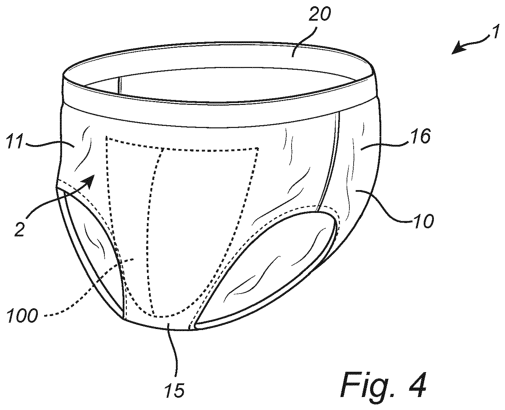

Figs. 1 and 4 show frontal perspective views of the washable absorbent underwear 1 as seen from the outer, garment-facing side 2. The absorbent underwear 1 in Fig. 1 is an incontinence pant for female incontinent persons and the absorbent underwear 1 in Fig. 4 is a pair of incontinence briefs for male incontinent persons. The absorbent underwear 1 in Figs. 1 and 4 have the same basic components, as described below, the main difference being the positioning of the absorbent assembly in the absorbent underwear 1.

Fig. 2 shows the underwear 1 of Fig. 1 from the inner, wearer-facing side 3 in a flat-out state with open side seams.

The absorbent underwear 1 comprises a textile pant 10 and includes an absorbent assembly 100 which is permanently secured in the textile pant 10, e.g., by being sewn onto the textile pant 10 and/or by being adhesively attached to the textile pant 10.

As shown in Fig. 2, the textile pant 10, the whole absorbent underwear 1 and the absorbent assembly 100 have an extension in a longitudinal direction y, in a transverse direction x and in a thickness direction z which is perpendicular to the longitudinal direction and the transverse direction. The absorbent underwear 1 has a front portion 11, a rear portion 16 and a crotch portion 15 bridging the front and rear portions 11,16.

The absorbent underwear 1 which is shown in Figs. 1 and 2 includes an elastic waistband 20 for providing improved retention of the absorbent underwear 1 on a wearer's body. An elastic waistband is an optional component of the absorbent underwear 1 as disclosed herein and may be applied as a separate component to the textile pant 10 of the absorbent underwear 1 or may be integrally formed with the textile pant 10, e.g., as a portion of the textile pant 10 having increased elasticity as compared to other parts of the textile pant 10. In the first case, the waistband may be any type of waistband as used in conventional underwear, such as a waistband made from cotton and elastane. In the latter case, the textile material in the waistband is typically the same as in other parts of the textile pant 10, such as cotton. A waistband which is integral with the textile pant 10 may be formed by folding over an edge portion of the textile pant 10 onto itself to form a waistband comprising two layers of pant material. Another way of producing a distinct integral waistband is by having a smaller textile gauge at the waist portion of the textile pant. The waistband may comprise additional elastic elements to provide enhanced elasticity at the waist portion of the textile pant.

The textile pant 10 may further include leg cuffs arranged around the leg openings of the pant. The leg cuffs may be elasticated leg cuffs comprising additional elastic elements providing enhanced elasticity at the leg openings.

The material of the textile pant 10 is typically a conventional underwear material such as a knitted (tricot) material and may include or consist of naturally derived fibers, man-made fibers, and mixtures of different fibers. Useful naturally derived fibers include cotton, wool, silk, cellulose, regenerated cellulose including rayon, viscose, modal, lyocell and tencel, bamboo, hemp, flax, ramie, coir, or banana.

Useful manmade synthetic fibers include polyamide, acrylic, polyester. As set out herein, the textile material may comprise a mixture or combination of naturally derived and/or synthetic fibers. The fibers may be recycled fibers. The textile material may comprise elastically stretchable material, e.g., elastane filaments, so that the absorbent underwear better can conform to the wearer's body and adapt to the wearer's movements. The elastic conformability of the textile pant contributes to preventing liquid from leaking out through the leg openings and to keep the absorbent assembly in place against the wearer's body. A textile material is generally breathable, allowing vapor to escape from the wearer's skin and from the absorbent assembly. Advantageously, the material in the textile pant is a combination of cotton and elastane.

The absorbent assembly 100 is integrated with the textile pant 10 and is positioned in a region of the textile pant 10 mostly likely to be exposed to micturition to provide absorbent capacity and leakage protection in a primary wetting area of the absorbent underwear 1. In the absorbent underwear 1 shown in Figs. 1 and 2, the absorbent assembly 100 is hourglass shaped and is positioned on a longitudinal centerline L and a transverse centerline T. The absorbent assembly 100 is present in the crotch portion 15 and has a front part extending into the front portion 11 of the absorbent underwear 1 and a rear part extending into the rear portion 16 of the absorbent underwear 1. With further reference to the example shown in Figs. 1 and 2, the absorbent assembly 100 has a generally hourglass shape with rounded front and rear edges 14, 17 and generally longitudinally extending concave side edges 18, 19. However, it is to be understood that the absorbent assembly 100 may have any useful shape within the scope of protection of the claims, such as the modified triangular shape shown in Fig. 4.

In the absorbent underwear 1 for male users which is shown in Fig. 4, the absorbent assembly 100 is located in an area of the front portion 11 of the absorbent underwear 1 extending in the longitudinal direction y from the waistband 20 towards the crotch portion 15. The absorbent assembly 100 has a generally triangular shape with a rounded lower corner positioned at the crotch portion 15 of the absorbent underwear 1. However, as disclosed herein, the absorbent assembly 100 may extend downward into the crotch portion 15 of the absorbent underwear 1 and may even extend through the crotch portion 15 and into the rear portion 16 of the absorbent underwear 1. It is to be understood that the absorbent assembly 100 may have any useful shape within the scope of protection of the claims.

As disclosed herein, the absorbent assembly 100 comprises several layers of different materials arranged stacked on each other. With reference to Fig. 3, the absorbent assembly 100 comprises a liquid permeable layer 120, a liquid impermeable layer 150 and an absorbent member 140 which is arranged between the liquid permeable layer 120 and the liquid impermeable layer 150. The absorbent member 140 comprises a liquid acquisition structure 141 of textile absorbent material and a liquid retaining structure 142 of textile absorbent material. It is to be understood that the liquid acquisition structure 141 of textile absorbent material and the liquid retaining structure 142 of textile absorbent material may each comprise two or more layers of material.

As set out herein, the liquid permeable layer 120 is a textile liquid permeable layer having a basis weight of 300 g/m² or less. The liquid retaining structure 142 is an absorbent component which may consist of a single layer of absorbent textile material or may comprise two or more individual layers of absorbent textile material.

The absorbent assembly 100 is shown in Fig. 3 to be permanently attached to the textile pant 10 in an attachment border 130 extending along the peripheral edges 16, 17, 18, 19 of the absorbent assembly 100. The attachment may comprise or consist of a seam through all layers of the absorbent assembly 100 which are present at the peripheral edges of the absorbent assembly 100. The attachment may further involve a sealing strip. The sealing strip may e.g., be adhesively applied to seal the edges of the material layers in the absorbent assembly 100. The sealing strip is liquid impermeable and preferably breathable and may be in the form of a polymer tape, such as a polyurethane tape.

In the embodiment shown in Fig. 3, all components in the absorbent assembly 100 are coextensive. However, this is not a requirement for the absorbent assembly 100 in washable absorbent hygienic underwear 1 as disclosed herein. Embodiments wherein the liquid acquisition structure 141 has a smaller area in the x-y plane than the liquid retention structure 142 may be useful as the liquid acquisition structure may then be arranged in a primary wetting area of the washable absorbent underwear with one or more absorbent layers of the liquid retention structure extending outside the peripheral edge of the liquid acquisition structure to create one or more security zones in secondary wetting areas of the absorbent assembly 100. Such secondary wetting areas may useful e.g., at the sides edges of a liquid acquisition structure 141 in the crotch portion 15 of absorbent hygienic underwear 1 as shown in Fig. 1, below a liquid acquisition structure 141 placed in the front portion 11 of absorbent hygienic underwear 1 as shown in Fig. 4 or outside an upper part of a liquid acquisition structure 141 of absorbent hygienic underwear 1 as shown in Fig. 4. The secondary wetting areas may be used to capture urine which overflows the liquid acquisition structure 141 at sudden gushes of urine as well as urine which misses the primary wetting area. This is a problem which is particularly common in washable absorbent hygienic underwear 1 for male users. The male anatomy makes it more difficult to predict where the primary wetting area will be. It has been found that a relatively large proportion of pant-type absorbent male absorbent incontinence products are wetted in the upper or waist portion of the front part of the incontinence product. Another problem particular to washable male absorbent underwear without superabsorbent material, is that urine which has initially been absorbed into an absorbent assembly 100 placed in the front portion of the absorbent underwear 1 as shown in Fig. 4, may flow downward into the crotch portion 15 of the absorbent underwear 1 under the influence of gravity. It may therefore be beneficial to arrange supplementary absorbent material in the crotch portion 15 of washable absorbent male underwear 1, to create an absorbent security zone in the crotch portion 15.

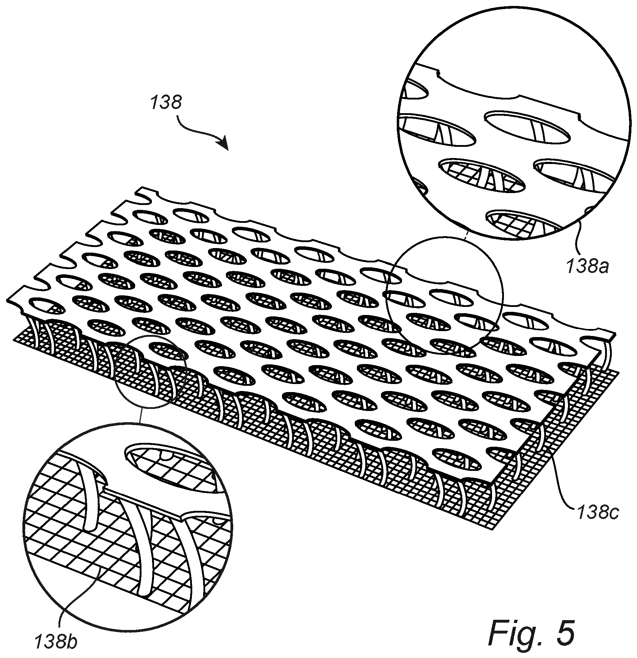

The liquid acquisition structure 141 comprises or consists of a spacer fabric 138 as shown in Fig. 5 where the liquid acquisition structure 141 is represented by a single layer of spacer fabric 138. As disclosed herein, the liquid acquisition structure 141 may contain further layers in addition to the spacer fabric layer, such as a second spacer fabric layer and/or a open-mesh material arranged between top layer 120 and the spacer fabric 130.

The spacer fabric 138 comprises a top layer 138a and a bottom web 138b and an interconnecting section 138c of pile filaments extending between the top web 138a and the bottom web 138b. The top web 138a and the bottom web 138b of the spacer fabric 138 may have different configurations to control the flow of the liquid in the spacer fabric 130 and liquid transfer to the liquid retaining structure 142. As disclosed herein, the top web 138a may have a lower density than the bottom web 138b. Furthermore, the density of the top web 138a and the bottom web 138b are higher than the density of the intermediate section 138c.

The liquid retaining capacity of the spacer fabric 138 is preferably considerably lower than the liquid retaining capacity of the liquid retaining structure 142. Accordingly, the retaining capacity of the spacer fabric may be 0.2 g/cm² or less and the retaining capacity in the liquid retaining structure 142 may be 0.3 g/cm² or more.

The density of the liquid retaining structure 142 is preferably considerably higher than the density of the spacer fabric 138 in the liquid acquisition structure 141.

The spacer fabrics 138 which are useful in a liquid acquisition structure 141 as disclosed herein have a density of 0.1 g/cm³ or less and a thickness of 2 mm or more and have a basis weight and a density which are lower than the basis weight and density of the liquid retaining structure 142.

A spacer fabric 138 is more compression resistant than other components that may be used for fluid flow control in absorbent articles but at the same time has a relatively low basis weight, such as 150 g/m² to 300 g/m² or 170 g/m² to 270 g/m². This allows for a slim and efficient fluid flow control member that will maintain its structure and fluid flow control properties when pressure is exerted on it during use of the washable absorbent hygienic underwear 1. As set out herein, the spacer fabric preferably has a bulk (ratio thickness/basis weight) of 10 cm³/g or more at an applied pressure of 5 kPa.

The number of pile filament connections per cm² and the dimension of the pile filaments makes the material soft but still able to withstand exerted pressures during its use. The pile filaments of the spacer fabric 138 has a high bending resiliency whereby the spacer fabric 138 expands immediately and almost completely to its original shape and thickness after removal of a compressive force exerted on the spacer fabric 138 during use.

The pile filaments in the intermediate section 138c of the spacer fabric 138 may together form channels, preferably in a diagonal direction of the fabric, creating an internal channel network promoting efficient distribution of liquid in the liquid acquisition structure 141.

The spacer fabric 138 is a highly porous material in which a free volume is present, also when the absorbent structure 140 is exposed to pressure exerted by a user wearing the absorbent male underwear 1, as a result of the high resistance to compression. Due to the free volume, the spacer fabric 138 can receive and temporarily hold a relatively large liquid volume. Thus, body liquids discharged into the absorbent assembly 100 can be effectively transferred from the liquid permeable layer 120 into to the spacer fabric 138 and can flow in the internal channel network in the intermediate section 138c of the spacer fabric 138 to be distributed over the area of the spacer fabric 138. The distributed liquid can then be transferred to the liquid retaining structure 142 over an area which may be as large as the area of the spacer fabric 138. The liquid which is transferred to the liquid retaining structure 142 is absorbed and retained in the absorbent material of the liquid retaining structure 142.

The top web 138a of the spacer fabric 138 may have an open structure to ensure rapid inflow and effective distribution of liquid in the spacer fabric 138. The spacer fabric 138 is preferably substantially free from absorbing fibers and does not contain superabsorbent material. However, the top web 138a and the bottom web 138b may contain hydrophilic fibers, such as regenerated cellulose fibers to promote wettability and liquid transfer between the liquid permeable layer 120 and the top web 138a and the bottom web 138b and the liquid retaining structure 142.

The spacer fabric 138 preferably comprises thermoplastic polymer fibres, preferably selected from but not limited to, polyesters, polyamides and polyolefins such as polyethylenes and polypropylenes, and may be a mixture or combination of any of these. The spacer fabric 138 may also advantageously comprise fibres from cotton and/or viscose. For a preferred spacer fabric 138, the yarns of the top and bottom webs 138a, 138b as well as the pile filaments 138c are of polyester.

The spacer fabric 138 may also contain surfactant to facilitate liquid penetration into the spacer fabric 138. It is also desirable that the spacer fabric 138 can be quickly drained from liquid by the liquid retaining structure 142, thus restoring free volume capacity for a next gush of urine.

The spacer fabric 138 is arranged in the absorbent assembly 100 with the top web 138a of the spacer fabric 138 facing and preferably in contact with the liquid permeable layer 120, and the bottom web 138b of the spacer fabric 138 is facing the liquid impermeable layer 150.

The liquid impermeable layer 150 is typically a polymer film layer or membrane and is preferably breathable, letting air and water vapor pass through but forming a barrier against liquid penetration. The liquid impermeable layer 150 may be directly attached to an absorbent layer, such as an absorbent layer in the liquid retaining structure 142. The attachment may be by a seam or by lamination. Lamination of the liquid impermeable layer 150 to the liquid retaining structure 142 may be made by means of a construction adhesive or by extruding the liquid permeable layer 150 onto the absorbent layer of the liquid retaining structure. The construction adhesive may be a polyurethane based adhesive.

### EXAMPLES AND DESCRIPTION OF TEST METHODS

### Example 1 - Density measurement

The weight and the thickness of the sample were measured. The thickness of the material was measured according to a standard test method NWSP120.6, option A, at 0.5 kPa pressure. The basis weight was calculated by dividing the weight with the area. Then, the density was calculated by dividing the basis weight with the thickness.

The density and basis weight have been measured on following material samples:

| Sample | Description | Density (g/cm³) | Basis weight (g/m²) |
|---|---|---|---|
| A | Spacer fabric | 0.06 | 247 |
| B | Spacer fabric | 0.06 | 175 |
| C | Spacer fabric | 0.08 | 230 |
| D | Ref spacer fabric | 0.13 | 258 |
| E | Ref spacer fabric | 0.18 | 400 |
| F | Abs retaining structure | 0.12 | 330 |
| G | Liquid permeable layer | 0.15 | 220 |

Sample A, B and C are spacer fabric materials from Muller Textil.

Sample D is a reference spacer fabric material from a washable absorbent underwear named Confitex Full Brief Extra, size M. Sample E is a reference spacer fabric from a washable absorbent underwear named ProTech Dry women, size M. Confitex Full Brief Extra, size M and ProTech Dry women, size M are commercially available washable absorbent underwear. Sample F is an absorbent retaining structure of terry material. Sample G is a liquid permeable layer of polyester.

### Example 2 - Compression resistance measurement

Different spacer fabric materials have been compression tested, to compare the compression resistance of spacer fabric in commercially available washable absorbent underwear with spacer fabrics of the present invention. Sample A, B and C are three spacer fabrics according to the invention. Sample D and E are two reference spacer fabrics as described in Example 1.

The principle of the method is to slowly compress a material with a metal rod to a force of 62 N while continuously measuring the thickness of the material. The result consists of the data points for force and extension. The force translates to a pressure given the contact area of the rod. The foot of the metal rod/piston has a diameter of 35 mm. The rod is mounted in a 100 N load cell in the upper fixture of an Instron testing apparatus. A flat plate is mounted in the bottom fixture and is centered under the rod so that a sample may be placed on top of the plate and be compressed without movement of the plate. The rate of movement of the rod is 10 mm per minute.

To test a sample, the rod is moved manually so that it is above the surface of the sample and the program is started. The rod moves down at a speed of 10 mm per minute until the limiting force is reached.

The sample has an area of 10 cm² and is punched from the material. The rod is pressed over the center of the sample and each sample is tested two times with 10 min between the first and second test to allow the material to recover.

The thickness of the spacer fabric A, B, C, D and E at different applied pressures were as following:

| Spacer fabric | 0.5 kPa | 5.0 kPa | 10 kPa | 20 kPa |
|---|---|---|---|---|
| A | 4.3 | 4.0 | 3.7 | 2.5 |
| B | 2.7 | 2.3 | 1.7 | 1.0 |
| C | 3.0 | 2.8 | 2.7 | 2.2 |
| D | 2.2 | 2.0 | 1.7 | 1.2 |
| E | 3.0 | 2.6 | 2.3 | 1.8 |

The bulk (cm³/g) of the spacer fabric A, B, C, D and E at different applied pressures were as following:

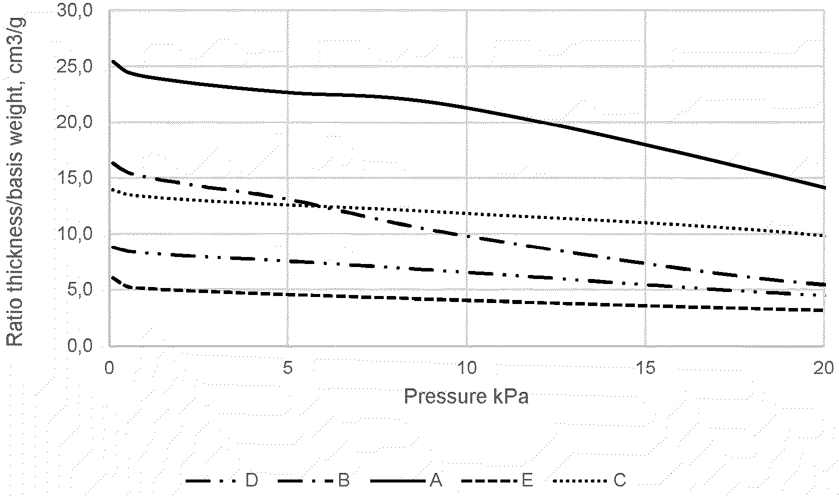

| Spacer fabric | 0.5 kPa | 5.0 kPa | 10 kPa | 20 kPa |
|---|---|---|---|---|
| A | 24.5 | 22.7 | 21.3 | 14.2 |
| B | 15.5 | 13.1 | 9.8 | 5.5 |
| C | 13.6 | 12.6 | 11.8 | 9.8 |
| D | 8.5 | 7.6 | 6.6 | 4.5 |
| E | 5.3 | 4.6 | 4.1 | 3.2 |

The result shows that the spacer fabric named A, B and C have a bulk of 10 g/cm³ or more, at an applied pressure of 0.5 k Pa, and at an applied pressure of 5 kPa, wherein the spacer fabric D and E have a bulk lower than 10 g/cm³ at an applied pressure of 0.5 k Pa and at an applied pressure of 5 kPa. The bulk value is the inversion of the density value, so a spacer fabric with a bulk of 10 cm³/g or more at an applied pressure of 0.5 k Pa and at an applied pressure of 5 kPa, has a density of less than 0.10 g/cm³ at an applied pressure of 0.5 k Pa and at an applied pressure of 5 kPa.

### Example 3 - Liquid retaining capacity measurement

The liquid retaining capacity test measures the amount of liquid held within a test sample after specified times of immersion and vertical drainage. The amount of test liquid that was retained by the test sample was used to calculate and report the liquid retaining capacity in g/cm². The test was performed in a climatized room controlled at 23 C and 50% RH.

The test procedure follows method WSP 010.1.R3 (20) part B (Liquid Absorptive Capacity) with modifications specified as follows. The test liquid is 0.9% saline solution. For the weighing part of the test, no cover glass was used as the test liquid is non-volatile. The sample size of the test sample was 3 cm by 5 cm and the dimension of the wire gauze was 4 cm by 6 cm. The sample was hanged vertically for 60 seconds.

Test samples were prepared as follows. Test samples were removed from a product by using scissors and then a punching tool was used to punch out the sample size of 3 cm by 5 cm.

The immersion and drainage procedure in the method NWSP10.1 was then followed with the modifications previously noted. The dry mass was subtracted from the wet mass and recorded as liquid mass absorbed in grams, then the liquid mass absorbed (g) was divided by the overall area (cm²) of the test sample. A total of three replicates were measured. The arithmetic mean for the liquid retaining capacity in g/cm² was then calculated.

Samples A, D and F were tested. Sample A was a spacer fabric from Muller Textil Sample D was a reference spacer fabric material from a washable absorbent underwear named Confitex Full Brief Extra, size M. Confitex Full Brief Extra, size M was a commercially available washable absorbent underwear. Sample F was an absorbent retaining structure of terry material.

The liquid retaining capacity (cm³/g) of the samples A, D and F were as following:

| Sample | Liquid retaining capacity (g/cm²) |
|---|---|
| A | 0.08 |
| D | 0.12 |
| F | 0.24 |

As can be seen from this result, the retaining capacity for sample A, which was a spacer fabric according to the present disclosure was below 0.1 g/cm² and the retaining capacity for sample D, which was a reference spacer fabric in the washable absorbent underwear named Confitex Full Brief Extra, size M, was above 0.1 g/cm². Furthermore, the sample F, which was an absorbent retaining structure of a terry material layer had a retaining capacity above 0.2 g/cm².

To increase the liquid retaining capacity further, the absorbent retaining structure of the present disclosure may comprise more than one liquid retaining layer, such for example two or more layers of the terry material according to sample F, wherein each of the layers have a liquid retaining capacity of 0.24 g/cm². The present disclosure may comprise two or more liquid retaining layers, such for example two or more layers of the terry material according to sample F.

### Example 4 - Acquisition measurement

The acquisition test measures the time required for an absorbent concept to absorb a specified amount of liquid. An acquisition cup was placed on the wearer facing side of the absorbent concept which is the wearer facing side of the liquid permeable layer. A dose of 10 ml is then added with a flow of 20 ml/seconds.

The acquisition time was measured on three absorbent concepts. Each of the absorbent concept 1, 2 and 3, comprising a liquid permeable layer, a liquid acquisition structure and a liquid retaining structure. So, each of the tested absorbent concept comprising a liquid permeable layer and an absorbent member.

The liquid permeable layer was Sample G in absorbent concept A, B and C. The width of the liquid permeable layer was 6 cm and the length of the liquid permeable layer was 18 cm. The liquid retaining structure was Sample F in concept A, B and C. The width of the liquid retaining structure was 6 cm and the length of the liquid retaining structure was 18 cm.

As liquid acquisition structure, the samples A, D and F were tested. The width of the liquid acquisition structure was narrower than the liquid permeable layer and the liquid retaining structure. The width of the liquid acquisition structure was 3.5 cm and the length of the liquid acquisition structure was 18 cm.

| Absorbent concept | Liquid acquisition structure | Inlet time (sec) |
|---|---|---|
| 1 | A | 2.0 |
| 2 | D | 3.7 |
| 3 | F | 5.2 |

As can be seen from this result, concept 1 provides a faster acquisition time than concept 2. So, an absorbent concept with a spacer fabric in accordance with the present invention (sample A) provides a faster inlet time compared to an absorbent concept with a reference spacer fabric (sample D).

It can also be seen that absorbent concept 1 and 2 with a spacer fabric provide a faster acquisition time than concept 3 having no spacer fabric. The absorbent concept 3 has an absorbent terry material as acquisition structure, so the same material as the liquid retaining structure.

## Claims

1. Washable absorbent assembly (100) adapted to be permanently or detachably secured in a textile pant (10) and having an extension in a longitudinal direction (y), in a transverse direction (x) and in a thickness direction (z) perpendicular to the longitudinal direction (y), and the transverse direction (x), and comprising:
- a liquid permeable layer (120),
- a liquid impermeable layer (150); and
- an absorbent member (140) located between the liquid permeable layer (120) and the liquid impermeable layer (150), the absorbent member (140) comprising a liquid acquisition structure (141) and a liquid retaining structure (142), the liquid acquisition structure (141) being arranged between the liquid permeable layer (120) and the liquid retaining structure (142), wherein
the liquid permeable layer (120) has a basis weight of less than 300 g/m²;
the liquid acquisition structure (141) comprises or consists of a spacer fabric (138) comprising a top web (138a), a bottom web (138b) and an intermediate section (138c) comprising pile filaments extending in the thickness direction (z) and connecting the top web (138a) with the bottom web (138b), the spacer fabric (138) having a density of 0.1 g/cm³ or less at an applied pressure of 0.5 k Pa, the liquid retaining structure (142) has a higher density than the liquid acquisition structure (141) and the washable absorbent assembly is devoid of superabsorbent particles.

2. Washable absorbent assembly (100) according to claim 1, wherein the liquid retaining structure (142) has a higher density than the spacer fabric (138) in the liquid acquisition structure.

3. Washable absorbent assembly (100) according to claim 1 or 2, wherein the spacer fabric (138) in the liquid acquisition structure (141) has a bulk of 10 cm³/g or more at an applied pressure of 5 kPa.

4. Washable absorbent assembly (100) according to any one of preceding claims, wherein the spacer fabric (138) in the liquid acquisition structure (141) has a bulk of 8 cm³/g or more at an applied pressure of 10 kPa.

5. Washable absorbent assembly (100) according to any one of the preceding claims, wherein the spacer fabric (138) in the liquid acquisition structure (141) has a bulk of 5 cm³/g or more at an applied pressure of 20 kPa.

6. Washable absorbent assembly (100) according to any of preceding claims, wherein the liquid retaining structure (142) has a higher basis weight than the liquid acquisition structure (141).

7. Washable absorbent assembly (100) according to any of preceding claims, wherein the spacer fabric has a basis weight between 150 g/m² and 300 g/m², or between 170 g/m² and 270 g/m².

8. Washable absorbent assembly (100) according to any of preceding claims, wherein the spacer fabric (138) has a thickness of 2 mm or more as determined using standard test method NWSP120.6, option A, at 0.5 kPa pressure.

9. Washable absorbent assembly (100) according to any of the preceding claims, wherein the liquid permeable layer has a basis weight of 250 g/m² or less, such as a basis weight of 200 g/m² or less or a basis weight of 150 g/m² or less.

10. Washable absorbent assembly (100) according to any one of the preceding claims, wherein the spacer fabric in the acquisition structure has a lower liquid retaining capacity than the liquid retaining structure.

11. Washable absorbent assembly (100) according to any one of the preceding claims, wherein the spacer fabric in the acquisition structure has a liquid retaining capacity of 0.2 g/cm² or less and the liquid retaining structure (142) has a liquid retaining capacity of 0.3 g/cm² or more.

12. Washable absorbent assembly (100) according to any one of the preceding claims, wherein the density of the liquid retaining structure (142) is 20% higher than the density of the spacer fabric (138), or more than 50% higher than the density of the spacer fabric (138).

13. Washable absorbent assembly (100) according to any one of the preceding claims, wherein the liquid acquisition structure (141) consists of a single layer of spacer fabric (138) or of a layer of spacer fabric (138) and one or more additional material layers.

14. Washable absorbent assembly (100) according to any one of the preceding claims, wherein the liquid retaining structure (142) consists of a single material layer or of 2 or more individual material layers.

15. Washable absorbent assembly (100) according to any one of the preceding claims, wherein the spacer fabric (138) of the liquid acquisition structure (141) is in direct contact with the liquid retaining structure (142).

16. Washable absorbent assembly (100) according to any one of the preceding claims, wherein the spacer fabric (138) of the liquid acquisition structure (141) is in direct contact with the liquid permeable layer (120).

17. Washable absorbent assembly (100) according to any one of the preceding claims, wherein a density of the bottom web (138b) of the spacer fabric (138) is higher than a density of the top web (138a) of the spacer fabric (138).

18. Washable absorbent assembly (100) according to claim 10, wherein the density of the top web (138a) of the spacer fabric (138) is higher than a density of the intermediate section (138c) of the spacer fabric (138).

19. Washable absorbent assembly (100) according to any one of the preceding claims, wherein the liquid retaining structure (142) is directly attached to the liquid impermeable layer (150), such as by being sewn or laminated to the liquid impermeable layer (150).

20. Washable absorbent assembly according to any of preceding claims, wherein the washable absorbent assembly is a washable absorbent pad adapted to be detachably secured to a textile pant.

21. Washable absorbent hygienic underwear (1), comprising an absorbent assembly (100) according to any of claims 1 to 19, and a textile pant (10), wherein the absorbent assembly is permanently secured in the textile pant (10) wherein the absorbent hygienic underwear (1) having an extension in a longitudinal direction (y), in a transverse direction (x) and in a thickness direction (z) perpendicular to the longitudinal direction (y), and the transverse direction (x), and the textile pant (10) comprising a front portion (11), a rear portion (16) and a crotch portion (15).

22. Washable absorbent hygienic underwear (1) according to claim 21, wherein the washable absorbent hygienic underwear is a washable absorbent incontinence underwear.

## Patentansprüche

1. Waschbare saugfähige Anordnung (100), die geeignet ist, um dauerhaft oder lösbar in einer Textilhose (10) befestigt zu werden, und die eine Ausdehnung in Längsrichtung (y), in Querrichtung (x) und in Dickenrichtung (z) senkrecht zur Längsrichtung (y) und zur Querrichtung (x) aufweist und Folgendes umfasst:
- eine flüssigkeitsdurchlässige Schicht (120),
- eine flüssigkeitsundurchlässige Schicht (150); und
- ein saugfähiges Element (140), das sich zwischen der flüssigkeitsdurchlässigen Schicht (120) und der flüssigkeitsundurchlässigen Schicht (150) befindet, wobei das saugfähige Element (140) eine Flüssigkeitsaufnahmestruktur (141) und eine Flüssigkeitsrückhaltestruktur (142) umfasst, wobei die Flüssigkeitsaufnahmestruktur (141) zwischen der flüssigkeitsdurchlässigen Schicht (120) und der Flüssigkeitsrückhaltestruktur (142) angeordnet ist, wobei
die flüssigkeitsdurchlässige Schicht (120) ein Flächengewicht von weniger als 300 g/m² aufweist;
die Flüssigkeitsaufnahmestruktur (141) ein Abstandsgewebe (138) umfasst oder daraus besteht, das eine obere Bahn (138a), eine untere Bahn (138b) und einen Zwischenabschnitt (138c) umfasst, der Florfasern umfasst, die sich in Dickenrichtung (z) erstrecken und die obere Bahn (138a) mit der unteren Bahn (138b) verbinden, wobei das Abstandsgewebe (138) eine Dichte von 0,1 g/cm³ oder weniger bei einem ausgeübten Druck von 0,5 kPa aufweist, die Flüssigkeitsrückhaltestruktur (142) eine höhere Dichte als die Flüssigkeitsaufnahmestruktur (141) aufweist und die waschbare saugfähige Anordnung frei von supersaugfähigen Partikeln ist.

2. Waschbare saugfähige Anordnung (100) nach Anspruch 1, wobei die Flüssigkeitsrückhaltestruktur (142) eine höhere Dichte als das Abstandsgewebe (138) in der Flüssigkeitsaufnahmestruktur aufweist.

3. Waschbare saugfähige Anordnung (100) nach Anspruch 1 oder 2, wobei das Abstandsgewebe (138) in der Flüssigkeitsaufnahmestruktur (141) ein Volumen von 10 cm³/g oder mehr bei einem ausgeübten Druck von 5 kPa aufweist.

4. Waschbare saugfähige Anordnung (100) nach einem der vorhergehenden Ansprüche, wobei das Abstandsgewebe (138) in der Flüssigkeitsaufnahmestruktur (141) ein Volumen von 8 cm³/g oder mehr bei einem ausgeübten Druck von 10 kPa aufweist.

5. Waschbare saugfähige Anordnung (100) nach einem der vorhergehenden Ansprüche, wobei das Abstandsgewebe (138) in der Flüssigkeitsaufnahmestruktur (141) ein Volumen von 5 cm³/g oder mehr bei einem ausgeübten Druck von 20 kPa aufweist.

6. Waschbare saugfähige Anordnung (100) nach einem der vorhergehenden Ansprüche, wobei die Flüssigkeitsrückhaltestruktur (142) ein höheres Flächengewicht als die Flüssigkeitsaufnahmestruktur (141) aufweist.

7. Waschbare saugfähige Anordnung (100) nach einem der vorhergehenden Ansprüche, wobei das Abstandsgewebe ein Flächengewicht zwischen 150 g/m² und 300 g/m² oder zwischen 170 g/m² und 270 g/m² aufweist.

8. Waschbare saugfähige Anordnung (100) nach einem der vorhergehenden Ansprüche, wobei das Abstandsgewebe (138) eine Dicke von 2 mm oder mehr aufweist, bestimmt unter Verwendung des Standardprüfverfahrens NWSP120.6, Option A, bei einem Druck von 0,5 kPa.

9. Waschbare saugfähige Anordnung (100) nach einem der vorhergehenden Ansprüche, wobei die flüssigkeitsdurchlässige Schicht ein Flächengewicht von 250 g/m² oder weniger aufweist, beispielsweise ein Flächengewicht von 200 g/m² oder weniger oder ein Flächengewicht von 150 g/m² oder weniger.

10. Waschbare saugfähige Anordnung (100) nach einem der vorhergehenden Ansprüche, wobei das Abstandsgewebe in der Aufnahmestruktur eine geringere Flüssigkeitsrückhaltekapazität als die Flüssigkeitsrückhaltestruktur aufweist.

11. Waschbare saugfähige Anordnung (100) nach einem der vorhergehenden Ansprüche, wobei das Abstandsgewebe in der Aufnahmestruktur eine Flüssigkeitsrückhaltekapazität von 0,2 g/cm² oder weniger aufweist und die Flüssigkeitsrückhaltestruktur (142) eine Flüssigkeitsrückhaltekapazität von 0,3 g/cm² oder mehr aufweist.

12. Waschbare saugfähige Anordnung (100) nach einem der vorhergehenden Ansprüche, wobei die Dichte der Flüssigkeitsrückhaltestruktur (142) 20 % höher als die Dichte des Abstandsgewebes (138) oder mehr als 50 % höher als die Dichte des Abstandsgewebes (138) ist.

13. Waschbare saugfähige Anordnung (100) nach einem der vorhergehenden Ansprüche, wobei die Flüssigkeitsaufnahmestruktur (141) aus einer einzigen Schicht Abstandsgewebe (138) oder aus einer Schicht Abstandsgewebe (138) und einer oder mehreren zusätzlichen Materialschichten besteht.

14. Waschbare saugfähige Anordnung (100) nach einem der vorhergehenden Ansprüche, wobei die Flüssigkeitsrückhaltestruktur (142) aus einer einzigen Materialschicht oder aus zwei oder mehr individuellen Materialschichten besteht.

15. Waschbare saugfähige Anordnung (100) nach einem der vorhergehenden Ansprüche, wobei das Abstandsgewebe (138) der Flüssigkeitsaufnahmestruktur (141) in direktem Kontakt mit der Flüssigkeitsrückhaltestruktur (142) steht.

16. Waschbare saugfähige Anordnung (100) nach einem der vorhergehenden Ansprüche, wobei das Abstandsgewebe (138) der Flüssigkeitsaufnahmestruktur (141) in direktem Kontakt mit der flüssigkeitsdurchlässigen Schicht (120) steht.

17. Waschbare saugfähige Anordnung (100) nach einem der vorhergehenden Ansprüche, wobei eine Dichte der unteren Bahn (138b) des Abstandsgewebes (138) höher als eine Dichte der oberen Bahn (138a) des Abstandsgewebes (138) ist.

18. Waschbare saugfähige Anordnung (100) nach Anspruch 10, wobei die Dichte der oberen Bahn (138a) des Abstandsgewebes (138) höher als eine Dichte des Zwischenabschnitts (138c) des Abstandsgewebes (138) ist.

19. Waschfähige saugfähige Anordnung (100) nach einem der vorhergehenden Ansprüche, wobei die Flüssigkeitsrückhaltestruktur (142) direkt an der flüssigkeitsundurchlässigen Schicht (150) befestigt ist, beispielsweise dadurch, dass sie an die flüssigkeitsundurchlässige Schicht (150) genäht oder laminiert ist.

20. Waschbare saugfähige Anordnung nach einem der vorhergehenden Ansprüche, wobei die waschbare saugfähige Anordnung ein waschbares saugfähiges Pad ist, das geeignet ist, um lösbar an einer Textilhose befestigt zu werden.

21. Waschbare saugfähige Hygieneunterwäsche (1), die eine saugfähige Anordnung (100) nach einem der Ansprüche 1 bis 19 und eine Textilhose (10) umfasst, wobei die saugfähige Anordnung dauerhaft in der Textilhose (10) befestigt ist, wobei die saugfähige Hygieneunterwäsche (1) eine Ausdehnung in Längsrichtung (y), in Querrichtung (x) und in Dickenrichtung (z) senkrecht zur Längsrichtung (y) und zur Querrichtung (x) aufweist und die Textilhose (10) einen vorderen Teil (11), einen hinteren Teil (16) und einen Schrittteil (15) umfasst.

22. Waschbare saugfähige Hygieneunterwäsche (1) nach Anspruch 21, wobei die waschbare saugfähige Hygieneunterwäsche eine waschbare saugfähige Inkontinenzunterwäsche ist.

## Revendications

1. Ensemble absorbant lavable (100) adapté pour être fixé de manière permanente ou amovible dans une culotte textile (10) et ayant une extension dans une direction longitudinale (y), dans une direction transversale (x) et dans une direction d'épaisseur (z) perpendiculaire à la direction longitudinale (y) et à la direction transversale (x), et comprenant :
- une couche perméable aux liquides (120),
- une couche imperméable aux liquides (150) ; et
- un élément absorbant (140) situé entre la couche perméable aux liquides (120) et la couche imperméable aux liquides (150), l'élément absorbant (140) comprenant une structure d'acquisition de liquide (141) et une structure de rétention de liquide (142), la structure d'acquisition de liquide (141) étant agencée entre la couche perméable aux liquides (120) et la structure de rétention de liquide (142), où
la couche perméable aux liquides (120) a un grammage inférieur à 300 g/m² ;
la structure d'acquisition de liquide (141) comprend ou consiste en un tissu d'espacement (138) comprenant une bande supérieure (138a), une bande inférieure (138b) et une section intermédiaire (138c) comprenant des filaments de poils s'étendant dans la direction d'épaisseur (z) et reliant la bande supérieure (138a) à la bande inférieure (138b), le tissu d'espacement (138) ayant une densité de 0,1 g/cm³ ou moins sous une pression appliquée de 0,5 kPa, la structure de rétention de liquide (142) a une densité supérieure à celle de la structure d'acquisition de liquide (141) et l'ensemble absorbant lavable est dépourvu de particules superabsorbantes.

2. Ensemble absorbant lavable (100) selon la revendication 1, où la structure de rétention de liquide (142) a une densité supérieure à celle du tissu d'espacement (138) dans la structure d'acquisition de liquide.

3. Ensemble absorbant lavable (100) selon la revendication 1 ou 2, où le tissu d'espacement (138) dans la structure d'acquisition de liquide (141) a un volume spécifique de 10 cm³/g ou plus sous une pression appliquée de 5 kPa.

4. Ensemble absorbant lavable (100) selon l'une quelconque des revendications précédentes, où le tissu d'espacement (138) dans la structure d'acquisition de liquide (141) a un volume spécifique de 8 cm³/g ou plus sous une pression appliquée de 10 kPa.

5. Ensemble absorbant lavable (100) selon l'une quelconque des revendications précédentes, où le tissu d'espacement (138) dans la structure d'acquisition de liquide (141) a un volume spécifique de 5 cm³/g ou plus sous une pression appliquée de 20 kPa.

6. Ensemble absorbant lavable (100) selon l'une quelconque des revendications précédentes, où la structure de rétention de liquide (142) a un grammage supérieur à celui de la structure d'acquisition de liquide (141).

7. Ensemble absorbant lavable (100) selon l'une quelconque des revendications précédentes, où le tissu d'espacement a un grammage compris entre 150 g/m² et 300 g/m², ou entre 170 g/m² et 270 g/m².

8. Ensemble absorbant lavable (100) selon l'une quelconque des revendications précédentes, où le tissu d'espacement (138) a une épaisseur de 2 mm ou plus, telle que déterminée en utilisant la méthode d'essai standard NWSP 120.6, option A, sous une pression de 0,5 kPa.

9. Ensemble absorbant lavable (100) selon l'une quelconque des revendications précédentes, où la couche perméable aux liquides a un grammage de 250 g/m² ou moins, tel qu'un grammage de 200 g/m² ou moins ou un grammage de 150 g/m² ou moins.

10. Ensemble absorbant lavable (100) selon l'une quelconque des revendications précédentes, où le tissu d'espacement dans la structure d'acquisition a une capacité de rétention de liquide inférieure à celle de la structure de rétention de liquide.

11. Ensemble absorbant lavable (100) selon l'une quelconque des revendications précédentes, où le tissu d'espacement dans la structure d'acquisition a une capacité de rétention de liquide de 0,2 g/cm² ou moins et la structure de rétention de liquide (142) a une capacité de rétention de liquide de 0,3 g/cm² ou plus.

12. Ensemble absorbant lavable (100) selon l'une quelconque des revendications précédentes, où la densité de la structure de rétention de liquide (142) est supérieure de 20 % à la densité du tissu d'espacement (138), ou supérieure de plus de 50 % à la densité du tissu d'espacement (138).

13. Ensemble absorbant lavable (100) selon l'une quelconque des revendications précédentes, où la structure d'acquisition de liquide (141) consiste en une seule couche de tissu d'espacement (138) ou en une couche de tissu d'espacement (138) et une ou plusieurs couches de matériau supplémentaires.

14. Ensemble absorbant lavable (100) selon l'une quelconque des revendications précédentes, où la structure de rétention de liquide (142) consiste en une seule couche de matériau ou en 2 couches de matériau individuelles ou plus.

15. Ensemble absorbant lavable (100) selon l'une quelconque des revendications précédentes, où le tissu d'espacement (138) de la structure d'acquisition de liquide (141) est en contact direct avec la structure de rétention de liquide (142).

16. Ensemble absorbant lavable (100) selon l'une quelconque des revendications précédentes, où le tissu d'espacement (138) de la structure d'acquisition de liquide (141) est en contact direct avec la couche perméable aux liquides (120).

17. Ensemble absorbant lavable (100) selon l'une quelconque des revendications précédentes, où une densité de la bande inférieure (138b) du tissu d'espacement (138) est supérieure à une densité de la bande supérieure (138a) du tissu d'espacement (138).

18. Ensemble absorbant lavable (100) selon la revendication 10, où la densité de la bande supérieure (138a) du tissu d'espacement (138) est supérieure à une densité de la section intermédiaire (138c) du tissu d'espacement (138).

19. Ensemble absorbant lavable (100) selon l'une quelconque des revendications précédentes, où la structure de rétention de liquide (142) est directement fixée à la couche imperméable aux liquides (150), par exemple en étant cousue ou stratifiée sur la couche imperméable aux liquides (150).

20. Ensemble absorbant lavable selon l'une quelconque des revendications précédentes, où l'ensemble absorbant lavable est un matelas absorbant lavable adapté pour être fixé de manière amovible à une culotte textile.

21. Sous-vêtement hygiénique absorbant lavable (1), comprenant un ensemble absorbant (100) selon l'une quelconque des revendications 1 à 19, et une culotte textile (10), où l'ensemble absorbant est fixé de manière permanente dans la culotte textile (10), où le sous-vêtement hygiénique absorbant (1) a une extension dans une direction longitudinale (y), dans une direction transversale (x) et dans une direction d'épaisseur (z) perpendiculaire à la direction longitudinale (y) et à la direction transversale (x), et la culotte textile (10) comprend une partie avant (11), une partie arrière (16) et une partie d'entrejambe (15).

22. Sous-vêtement hygiénique absorbant lavable (1) selon la revendication 21, où le sous-vêtement hygiénique absorbant lavable est un sous-vêtement d'incontinence absorbant lavable.
